# EUROPEAN PATENT SPECIFICATION

(11) **EP 1 165 151 B1**
(45) Date of publication and mention of the grant of the patent: **03.01.2007**
(21) Application number: 00923201.8
(22) Date of filing: 11.04.2000
(51) Int. Cl.: A61L 2/28, G01N 31/22

(54) **INDICATORS FOR MONITORING STERILIZATION WITH PLASMA**
INDIKATOREN ZUR ÜBERWACHUNG VON PLASMASTERILISATIONEN
INDICATEURS DESTINES AU CONTROLE DE LA STERILISATION AU PLASMA

(30) Priority: 13.04.1999 US 129130 P
(43) Date of publication of application: 02.01.2002
(73) Proprietor: Patel, Gordhanbhai N., Middlesex, NJ 08846 (US)
(72) Inventor: Patel, Gordhanbhai N., Middlesex, NJ 08846 (US)
(74) Representative: Winter, Brandl, Fürniss, Hübner Röss, Kaiser, Polte Partnerschaft Patent- und Rechtsanwaltskanzlei
(86) International application number: PCT/US2000/009493
(87) International publication number: WO 2000/061200

(56) References cited:
- WO-A-98/52621
- WO-A-98/58683
- US-A- 4 407 960

## Description

### BACKGROUND OF THE INVENTION

### 1. FIELD OF THE INVENTION

The present invention relates to devices comprising chemical indicators for monitoring plasmas, in particular for sterilization of medical supplies with in particular that from hydrogen peroxide.

### 2. BRIEF DESCRIPTION OF PRIOR ART

A wide variety of medical supplies and biological wastes are sterilized with materials and techniques, such as steam, ethylene oxide (ETO), high energy radiation and plasma. It is essential to assure that these supplies or wastes are actually sterilized. A number of indicators, dosimeters and monitors have been proposed in the literature. They include biological and chemical indicators. The color changing chemical indicators are inexpensive and hence are widely used.

In order to assure the sterilization with plasma has taken place, the indicator must determine integral value of parameters, such as time, temperature, humidity, and concentration of the plasma gas. Biological indicators made from cultures, such as Bacillus subtilis spores, bacillus pumilus spores and clostridium sporogenes spores have been used for monitoring the sterilization, for example, US patents 5,801,010, 5,788,925, and 5,866,356. However, chemical indicators are preferred because they are simple and inexpensive.

WO9846994A1 and WO9846279A1 describe compositions comprising dyes and organic amines which change color when contacted with hydrogen
peroxide plasma. WO9852621A1 describes dyes, such as, acid fuschin which change color when contacted with hydrogen peroxide, vapour.

WO 98/58683 discloses a detection device having an enclorsure providing a air gap between the detector substate and the enclosure. Since the device uses a paper substrate it is unsuitable for use with plasmas

### SUMMARY OF THE INVENTION

There is provided a device for monitoring comprising: at least one layer of polymer exposable to said plasma, having incorporated therein
a) an indicator capable of undergoing at least one color change
b) an activator for said indicator wherein said activator, when contacted with said plasma, undergoes a reaction wherein the product of said reaction causes said indicator to undergo said color change.

The indicators suitable for use in this device include pigments, dyes, precursors of said dyes, and mixtures of any of these. Suitable indicators include pH-sensitive sensitive dyes such as phenol red, m-cresol purple, pararosaniline or mixtures thereof. A desirable quality of the indicator is the ability to undergo halogenation, especially bromination or oxidation. The source of such brominating or oxidizing agents being the reaction between the plasma and the activator. Desirably the indicator undergoes a yellow-to-blue, red-to-yellow or red-to-blue color change.

The polymer used in the device is, suitably, soluble in water or dispersible in an aqueous medium solvent . A broad dass of polymers may be used. They may be homopolymers, copolymers or a mixture thereof, suitably a polymer of styrene, acrylate, acrylic acid, acrylamide, vinyl acetate, vinyl alcohol, vinyl chloride, styrene, polyurethanes, cellulose nitrate, carboxymethyl cellulose or a mixture thereof. Desirably, the polymer is an acrylate polymer, cellulose nitrate or carboxymethylcellulose

Suitably the reaction product of the activator and the plasma is a halo-acid. Suitable activators are salts such as halides, preferably bromides. Desirable halides include bromides of alkali metals or of quaternary amines such as tetrabutylammonium bromide or tetraethylammonium bromide or a mixture thereof. The activator may also be a salt of an amine and an organic or inorganic acid. It may also be a metal thiocyanate such as sodium thiocyanate .

The device may additionally comprise an additive to control the diffusion of plasma gases, such a crosslinking agent or a plasticizer. These may include zinc compounds or polyaziridines.

The device may have two layers, that is to say additionally comprising a polymeric top layer. This may be a wedge shaped polymeric top layer.

The process of making a device of the present invention comprises dissolving or dispersing the components thereof in a solvent therefore, applying the thus formed solution or dispersate to a substrate and permitting the solvent to evaporate.

The substrate may be a container for an item to be sterilized. It may also be a plastic film, or metal, including but not limited polyester film or spun bonded polyolefins.

In a desirable embodiment of the invention the solution is an ink formulation suitably an aqueous ink formulation most suitably one which comprises an acrylate polymer.

A process of using a device of the present invention for monitoring sterilization of materials comprises the steps of affixing the device to said materials or containers containing same, carrying out the process of sterilization including the step of introducing the plasma into a vessel containing said materials or containers thereof and observing the presence of a color change of said device.

Among the plasmas which may be utilized in this process are those derived from hydrogen peroxide, perchloric acid and oxygen, most suitably from hydrogen peroxide.

### DETAILED DESCRIPTION OF THE DRAWINGS

Figure 1. A side schematic cross section of one embodiment of the plasma sterilization indicator of the invention where an indicator layer comprised of a polymeric binder, plasma activator and plasma indicator is applied on a substrate.
Figure 2. A side schematic cross section of the plasma sterilization indicator of the invention having an adhesive layer and a release layer.
Figure 3. A side cross section of a two layered plasma sterilization indicator layer.
Figure 4. A side schematic cross section of the moving boundary plasma sterilization indicator created by coating a wedge shaped barrier on the plasma indicator.

### DESCRIPTION OF THE PREFERRED EMBODIMENTS

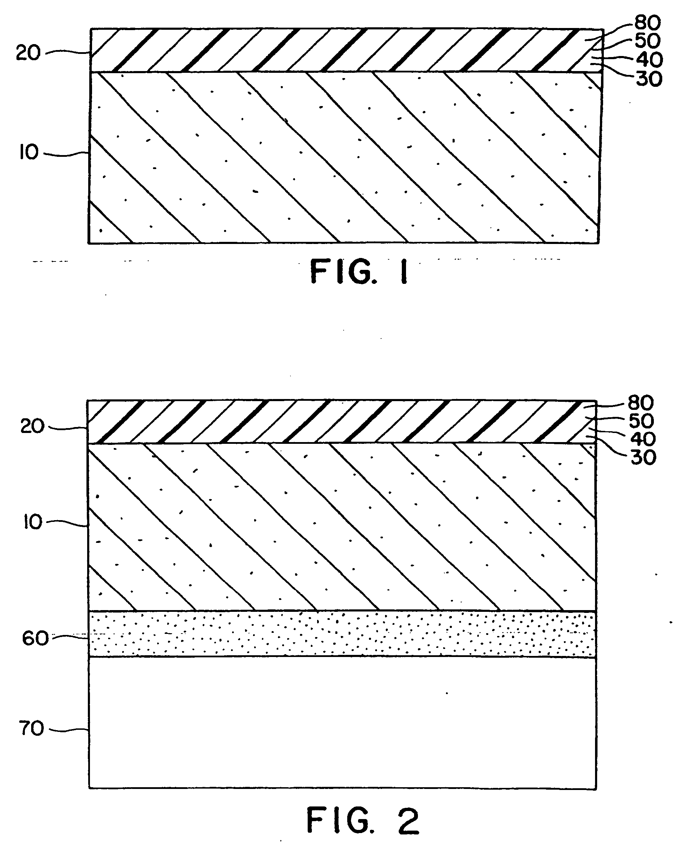

The device can be best described by reference to the Figures. As shown in Figure 1, the device in its simplest form is comprised of an indicator layer 20, applied on a substrate 10. The substrate 10 can also be a container, such as pouch for products to be sterilized. The indicator layer 20 is composed of a polymer 50 containing at least one indicator 30 capable of undergoing a color change when reacted with a reactive species produced by exposure of the activator 40 to plasma. The layer 20 may additionally contain other additives 80 to control the rate of the color change, such as a crosslinking agents or plasticizers to control diffusion of plasma gases either to increase or decrease the penetration of plasma.

As shown in Figure 2, the substrate 10 of the device can further comprise an adhesive layer 60. The adhesive layer allows the device to be affixed to a container of product to be sterilized. To the bottom of the adhesive layer 60, can further be affixed a release layer 70 for ease in packaging and for removal just prior to use.

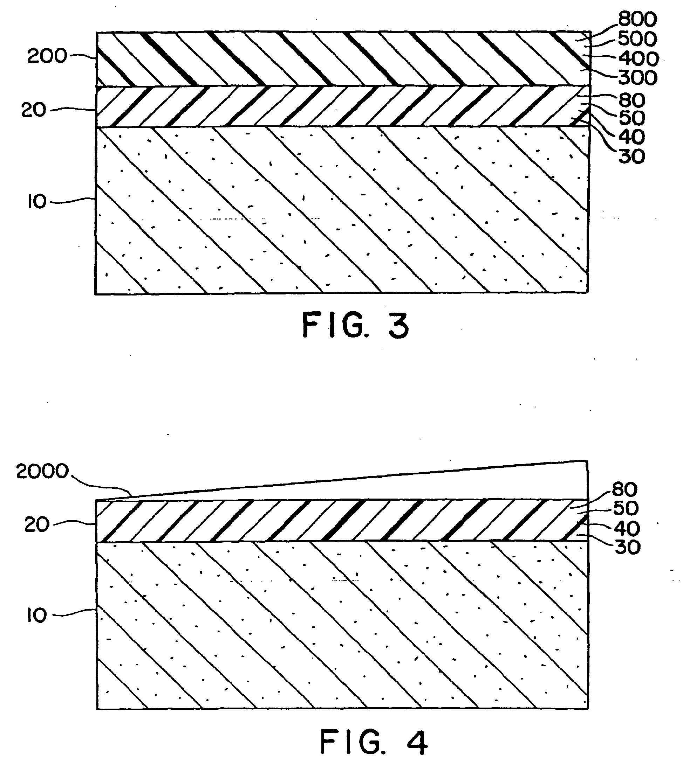

As shown in Figure 3, a simple form of a multi-layer device can have two indicator layers 20 and 200. The multi-layer device can thus undergo more than one color change. The device can be created by applying another indicator layer 200 over the first indicator layer 20. The indicator 300 of the indicator layer 200 would be different from indicator 30 of the indicator layer 20. The polymers 50 and 500, additives 80 and 800 and the activators 40 and 400 of the first indicator layer 20 and the second indicator layer 200 could be the same or different. When exposed to plasma, the top indicator layer 200 may undergo a color change (e.g., red-to-colorless) first followed by the color change of the bottom indicator layer 20 (e.g., colorless-to-blue). Thus the device would undergo two color changes. There are many variations of the multi-layer device. For example, the device can have more than two indicator layers and can have one or more barrier layers between or on them.

A simple form of a moving boundary indicator device is shown in Figure 4. The moving boundary device can be created by applying a wedge shaped barrier layer 2000 over the indicator layer 20. The barrier layer will resist but will be permeable to plasma and its components. There are many variations to this moving boundary device. For example, the wedge barrier coat may contain indicator and both the indicator and the barrier layers be in form of wedge but in opposite direction. Other variations of the plasma indicator device are also possible, for example, a gradient device can be created by coating a series of formulations having the time required for the color change either increase or decrease. Such gradient can be obtained by coating such formulations in form of lines or bars next to each other.

The device may also include a printed number, an image, a bar code or a message, e.g., "if this print is green, the product inside is sterilized".

Activators produce other reactive species, such as those from compounds having: -I, -Br, -Cl, -NH-, -NH₂, -OH, =O, -NO, -NO₂, -NO₃, -SO₃, -SO₃H, -COOH, -PO₄, SCN, etc. Especially desirable are bromides, i.e., from tetraethyl ammonium bromide (TEAB), add Br as an oxochrome to the dyes. Similarly other activators add other oxochromes to the dye.

Results from a series of experiments indicate the product formed by the reaction of phenol red and the bromide salts is most likely bromophenol blue. We have also found that nature, e.g., pH, of the medium i.e., binder, plays an important role in determination of color of the reaction product, especially the dye produced is a pH-sensitive dye. For example, when exposed to hydrogen peroxide, phenol red changes from yellow-to-orange red in polyvinyl alcohol while it changes from yellow-to-blue in EC001270 (an acrylate printing ink extender supplied by Environmental Inks and Coating Co., Lithicum, MD). The pH of EC001270 coating is about 4-5 while that of polyvinyl alcohol is about 7. Bromophenol blue appears red in polyvinyl alcohol while blue in EC001270.

The color change in EC001270 and TEAB system for chlorophenol red is yellow-to-purple, m-cresol purple is yellow-to-blue, cresol red is yellow-to-purple, phenol red is yellow-to-blue, and thymol blue is yellow-to-blue upon exposure to hydrogen peroxide vapor and its plasma. Evaluation of these and other dyes which develop colors indicates that brominated dyes are formed for some of these dyes upon exposure to hydrogen peroxide and its plasma.

As shown in Table 1 below, the conclusion that EC001270, TEAB and dyes which develop color is supported by the pH range and colors of the substrate dyes (dyes before the treatment) and corresponding brominated dyes (after exposing to hydrogen peroxide and its plasma).

**Table 1. Color of the starting substrate dyes and corresponding brominated dyes at low and high pH ranges.**

| Substrate dye | Low pH | Color | High pH | Color | Brominated dye | Low pH | color | High pH | Color |
|---|---|---|---|---|---|---|---|---|---|
| Chlorophenol red | 4.8 | Yellow | 6.4 | Red | Bromochlorophenol red | 3.2 | yellow | 4.8 | Purple |
| m-Cresol purple | 7.4 | Yellow | 9.0 | Purple | Bromocresol green | 3.8 | yellow | 5.4 | Blue-green |
| Cresol red | 7.2 | Yellow | 8.8 | Purple | Bromocresol purple | 5.2 | yellow | 6.8 | Purple |
| Phenol red | 6.8 | Yellow | 8.2 | Red | Bromophenol blue | 3.0 | yellow | 4.6 | Blue |
| Thymol blue | 8.0 | Yellow | 9.2 | Blue | Bromothymol blue | 6.0 | yellow | 7.6 | Blue |
| Xylenol blue | 8.0 | Yellow | 9.6 | Blue | Bromoxylenol blue | 6.0 | yellow | 7.6 | Blue |

**Indicators:** Any material which undergoes a color change in presence of a plasma activator can be used as a plasma indicator. Plasma indicators are also referred herein to as indicators. Most preferred classes of plasma indicators are dyes, pigments and their precursors. A large number of dyes and dye precursors, e.g., are listed in Table 2.

### Table 2. List of useable dyes.

These have been tested with tetrabutylammonium bromide as an activator in polyacrylate (EC001270) and in the presence of solvent based cellulose nitrate.

Acid alizarin violet N, acid black 24, acid black 48, acid blue 113, acid blue 120, acid blue 129, acid blue 161, acid blue 25, acid blue 29, acid blue 40, acid blue 41, acid blue 45, acid blue 80, acid blue 93, acid fuschin, acid green 25, acid green 27, acid green 41, acid orange 74, acid red 1, acid red 114, acid red 151, acid red 88, acid violet 17, acid violet 7, acid yellow 99, acridine orange, acridine orange base, acridine orange G, acridine yellow G, acriflavine hydrochloride, alcian blue BGX, alcian yellow, alizarin, alizarin blue black SN, alizarin complexone, alizarin complexone dihydrate, alizarin red, alizarin violet 3R, alizarin yellow GG, alizarin yellow R, alkali blue 6B, alkali fast green 10GA, alphazurine A, aluminon, aminoacridine hydrochloride, aminoanthraquinone, aminophthalhydrazide, aniline blue, astra blue 6GLL, auramine O, azocarmine, azocarmine B, azure A, azure B, azure B thiocyanate, azure C, basic blue 3, basic blue 41, basic blue 66, basic fuchsin, basic red 29, basic yellow 11, benzo purpurin 4B, biebrich scarlet NA salt, bismarck brown B, bismarck brown Y, blue tetrazolium, bordeaux R, brilliant blue B, brilliant blue G, brilliant cresyl blue ALD, brilliant crocein MOO, brilliant green, brilliant sulphaflavine, brilliant yellow, bromochlorophenol blue, bromocresol green, bromocresol purple, bromophenol blue, bromopyrogallol red, bromothymol blue, bromoxylenol blue, calmagite, carbol fuchsin, carminic acid, carotene, celestine blue, Chicago sky blue, chlorophenol red, chrome azurol S, chromotrope 2B, chromotrope 2R, chromoxane cyanine B, chrysoidin, chrysophenine, cibacron brilliant red 3BA, Congo red, copper(II) phthalocyanine, cresol purple, cresol red, cresol, cresolphthalein, cresolphthalein complexone, crystal violet, curcumin, darrow red, diaminoacridine hemisulfate, diazo red RC, dibromofluorescein, dichlorofluorescein, dichloroindophenol, dicinnamalactone, diethylaminomethyl coumarin, diethyloxacarbocyanine iodide, diethylthiatricarbocyanine iodide, dihydroxy benzenesulfonic acid, dilithium phthalocyanine, dimethyl methylene blue, dimethylglyoxime, dimethylindoaniline, dinitro diphenylamine, diphenylthiocarbazone, direct blue 71, direct green 6, direct red 23, direct red 75, direct red 81, direct violet 51, direct yellow 62, disodium phthalocyanine, disperse blue 14, disperse blue 14, disperse blue 3, disperse orange, disperse orange 11, disperse orange 25, disperse yellow 7, emodin, eosin B, eosin Y, eriochrome black T, eriochrome blue black B, erioglaucine, erythrosin B, ethyl eosin, ethyl orange, ethyl red, ethyl violet, Evans blue, fast black, fast blue B salt, fast blue BB, fast blue RR, fast blue RR salt, fast corinth V salt, fast garnet GBC base, fast green FCF, fast red aluminum salt, fast red violet LB salt, fast violet B salt, fat brown RR fat green GDC salt, flavazin I, fluorescein, fluorexon, gallocyanine, guinea green B, hematoxylin, hydroxy naphthol blue, 1,4-hydroxynaphthoquinone, indigo, indigo carmine, indoline blue, iron(II) phthalacyanine, janus green B, lacmoid, leishman stain, leuco crystal violet, leucomalachite green, leucoquinizarin, light green SF yellowish, lissamine green B, litmus, luxol fast blue, malachite green base, malachite green hydrochloride, malachite green oxalate, metanill yellow, methyl eosin, methyl green, methyl orange, methyl red, methyl violet 2B, methyl violet B base, methyl yellow, methylene blue, methylene green, methylene violet 3RAX, methylesculetin, methylthymol blue, mordant blue 9, mordant brown 24, mordant brown 4, mordant orange, mordant orange 1, mordant orange 6, mordant red 19, mordant yellow 10, morin hydrate, murexide, naphthochrome green, naphthol AS, naphthol blue black, naphthol green B, naphthol yellow, naphtholbenzein, naphtholbenzene, naphtholphthalein, neutral red, new coccine, new fuchsin, new methylene blue N, nigrosin, nile blue A, Nile blue chloride, nitrazine yellow, nitro red, nitro-phenanthroline, nitrophenol-2, nitrophenol-3, nitrophenol-4, nitrophenylazo-resorcinol, nuclear fast red, oil blue N, oil red EGN, oil red O, orange G, orange II, palatine chrome black 6BN, palatine fast yellow BLN, pararosaniline acetate, pararosaniline base, pararosaniline chloride, patent blue VF, pentamethoxytriphenylmethanol, phenanthroline, phenazine, phenol red, phenolphthalein, phenolphthalein diphosphate, phenothiazine, phenylazoaniline, phenylazodiphenylamine, phenylazoformic acid, phenylazophenol, phloxine B, phthalocynine, pinacyanol chloride, plasmocorinth, ponceau S, primuline, procion red MX-5B, procion yellow H-E3G, prussian blue, purpurin, pyridlazo naphthol, pyridylazoresorcinol sodium salt, pyrocatechol violet, pyrogallol red, pyronin B, quinaldine red, quinizarin, quinoline yellow, reactive black 5, reactive blue 15, reactive blue 2, reactive blue 4, reactive orange 16, resazurin, resorcin crystal violet, rhodamine B, rhodamine B base, rhodamine GG, rhodamine S, rhodanine, rosalic acid, rose bengal, rose bengal iactone, safranine O, solvent blue 35, solvent blue 59, solvent green 3, styryl 7, sudan black B, sudan orange G, sudan red 7B, sulfobromophthalein sodium salt, sulforhodamine B, tartrazine, tetrabromophenol blue, tetrabromo phenolphthalein, tetrabromo phenolphthalein, tetraiodo phenolphthalein, tetraphenyl-butadiene, tetrazolium violet, thiazol yellow G, thioflavin S, thioflavin T, thionin, thymol blue, thymolphthalein, thymolphthalein monophosphate, thymolphthalein monophosphate, toluidine blue O, triphenylmethyl bromide, tropaelin O, trypan blue, turmeric, vanillin azine, variamine blue RT salt, variamine blue RT salt, victoria blue B, victoria blue B, victoria pure blue BO, wright stain, xilidine ponceau 2R,, xylenol blue, and xylenol orange.

Some dyes, e.g., malachite green, reduced with ascorbic acid, sodium sulfite and formaldehyde were also used. Some of these dyes are fluorescence dyes and they either lost their fluorescence or there was a change in fluorescence.

**Simultaneous reactions:** We have also observed that certain dyes, e.g., phenol red show development of color (due to formation of bromophenol blue) followed by de-coloration upon prolonged exposure to hydrogen peroxide or plasma treatment.

**Activators:** Any chemical which produces reactive species with a plasma or hydrogen peroxide which, when reacted with a indicator compound, degrade, cleave or add to the molecule or react to form a colored compound can be used as an indicator activator. Similarly, a chemical which when reacted with plasma or hydrogen peroxide and produces a chemical which changes environment, e.g., pH of the medium, can also be used as an indicator activator. Production of an acid or base introduces a change of a pH-sensitive dye. A variety of classes of organic and inorganic compounds may be used as activators for monitoring hydrogen peroxide and its plasma. They include alcohols, amides, amines, bisulfites, bisulfates, carbonates, carbamates, chelates, metal complexes, cyanates, esters, halides, halocarbons, ketones, nitrites, nitrates, nitriles, nitro, nitroso, oximes, phenols, phosphates, sulfates, sulfides, sulfites, thiocyanates, ureas, urethanes, salts, oxidants and reducing agents. Organic and inorganic salts, especially halides were very effective activators. The specific examples of compounds explored as plasma activators with some selected dyes (e.g., chrome azurole S, methyl green, acid fuschin, direct blue 71 and nile blue) are listed in Table 3.

### Table 3. List of specific examples of activators.

Abietic acid, acetone oxime, aluminum acetylacetonate, aluminum ammonium sulfate, aluminum chloride, aluminum sulfate, amino deoxy d-sorbitol, ammonium acetate, ammonium bisulfite, ammonium bromide, ammonium carbamate, ammonium nitrate, ammonium sulfamate, ammonium sulfite, ammonium thiocyanate, ammonium thiosulfate, ascorbic acid, azodicarbonamide, azodicarbonamide, benzilic acid, benzoic acid, benzophenone, benzophenone tetracarboxylic acid, benzophenonetetracarboxylic diahydride, benzoquinone dioxime, benzoquinone dioxime, benzyloxy)phenol, butyl phenol, caffeine, calcium ferrocyanide, catechol, catechol, chloranilic acid, copper thiocyanate, cupferron, cupferron, cyclopenatanone oxime, dehydroacetic acid, di-butyl-t-4-methylphenol, dihydroxy acetophenone, dihydroxy dimethoxy benzophenone, dihydroxy naphthalein disulfonic acid, dihydroxyacetophenone, dihydroxy-dimethoxybenzophenone, dimethyl fumarate, dimethyl tartrate, diphenyl butyro lactone, diphenylglyoxime, diphenylthiocarbazone, di-t-butyl-4-methylphenol, dithizone or diphenylthiocarbazone, ethylcarbonate, ethylenediamine tetraaceticacid and ts salts, ferroin, fumaric acid, gallic acid, gluconic acid fe(ii) salt, glucose penta acetate, glucose penta acetate, glucose pentaacetate, glutaaric acid, glycerophosphate, glyconolactone, hexahloro norborene dicarboxylic acid, hydroquinone, hydroxy acetophenone, hydroxy acetophenone, hydroxy cinnamic acid, hydroxy methoxybenzophenone, hydroxy octyloxy benzophenone, hydroxybenzophenone, hydroxybenzophenone, hydroxymethoxybenzophenone, hydroxyquinoline, hydroxyquinoline, inositol, iron acetylacetonate, iron complexes such as potassium ferrocyanide, iron sulfate, isoascorbic acid, levulinic acid, maleic acid, maleic acid, malic acid, mandelic acid, mercaptobenzothiazole, methyldinitrosalicilate, methyldinitrosalicylate, methylesculetin, methyltrihydroxybenzoate, naphthol, naphthol-disulfonic acid, naphthoquinone tetrasulfate sodium salt, nitron, nitron, nitroso-1,2-naphthol, nitrosophenol, oxalic acid, phenanthroline, phenanthroline, phthalide, propylgalliate, propylgalliate, pydine aldoxime, pyruvic acid, resorcinol, rutin hydrate, salicyladoxime, salicyladoxime, salicylanamid, salicylanilide, salicylic acid, sodium acetylacetonate, sodium bisulfite, sodium cyante, sodium diethyldithio carbamate, sodium diethyldithiocarbamate, sodium dithionite, sodium hydrosulfide, sodium nitrite, sodium persulfate, sodium sulfite, sodium sulfite, sodium sulfite, sodium sulfite, sodium thiocyanate, sodium thiocyanate, sodium thiosulfate, sulfosalicyclic acid 5, tannic acid, tetrabutylphosphonium bromide, tetrahydroxybenzophenone, tetrahydroxybenzophenone, tetramethylhexane diamine, tetronic acid, tetronic acid, thiodiglycolic acid, thiodipropionic acid, thioglycolic acid, thiourea, thiourea, tribenzylamine, trichloroacetamide, trichlorobenzylacetate, trihydroxybenzophenone, trihydroxybenzophenone, urea, vitamin-c, and vitamin-c palmitate

**Effect of halides:** Many organic and inorganic halides are highly sensitive indicator activators. These halides include, acetyl choline chloride, ammonium bromide, choline chloride, choline iodide, dodecyltrimethylammonium bromide, glycidil trimethyl ammonium chloride, potassium bromide, potassium iodide, sodium iodide, tetrabutyl ammonium iodide, tetraethyl ammonium bromide, tetrahexyl ammonium bromide, tetramethyl ammonium chloride and tetrabutyl phosphonium bromide: Bromides were more effective than other halides.

Acid base salts, e.g., those produced by neutralizing, primary, secondary and tertiary amines (e.g., hexyl amine, diethanolamine, tetramethylhexane diamine) with acids such as acetic acid, hydrochloric acid, hydrobromic acid and hydroiodic acid are also effective activators.

The time required for the color change can be controlled by using a proper mixture of the activators/halides/bromides, in order to minimize effect of ethylene oxide lower concentration of halides is preferred for certain dyes.

**Effect of concentration of** a**ctivator:** With certain dyes as low as 0.5% of activator is effective in introducing noticeable color change with plasma. The rate of color change increases with increasing the concentration of the activator. High concentrations of activator sometimes lead to de-coloration of the colored formed. 0.5 to 50 w/w% concentration of a activator may be useful. The preferred concentration range is 2-10 w/w% of the total solid.

**Polymers:** A matrix or a medium in which a wide variety of organic and inorganic polymeric materials can be used as carrier for the indicator as long as the activators and indicators can be dissolved or dispersed in them. Water-soluble, water-dispersible and water-insoluble polymers may be used. It is preferable to use water-soluble and water-dispersible polymers. These can be formulated in the form of ink formulations, such as flexo and gravure inks. Other inks such as those for letter press, offset and screen printing can also be used. Selection of a polymer depends upon the printing/coating equipment to be used.

Usually acrylic polymers, emulsion of acrylic polymers, occasionally natural polymers, such as starches, lignins, and lignin derivatives are used in inks. Resins are water soluble or emulsifiable through neutralization with basic compounds, such as ammonia and amines. Inks contain a variety of additives to eliminate foaming, dispersion of pigments, rheological modifiers, and slip agents.

Polymers used in inks include homopolymers, copolymers and blockcopolymers including those of ethylene acrylic acid, ethylene methacrylic acid, ethylene n-butyl acrylate, and ethylene methyl acrylate. The polymers used in the inks could also be a mixture of homo and copolymers, e.g., those of methylmethacrylate, acrylic acid, styrene, methyl acrylate, other esters may include crosslinking agents, such as polyvinylazaridines metal salts and complexes, e.g., that of zinc.

Commercial sources for suitable polymers for ink formulations include Air products (Allentown, PA), Rohm and Haas (Philadelphia, PA), S.C. Johnsons and Sons (Racine, WI), Witco (Houston, PA) and ESI (Valley Stream, NY). Though a large number of polymers are suitable an ink extender, EC001270 made by Environmental Inks and Coating Co., Lithicam, MD which is composed about 40% styrene-acrylic polymers, a few percent ammonium hydroxide, additives, such as wax and alcohol and the balance water, has been found very suitable.

The nature of the polymer, e.g., pH and permeability to the gases, plays an important role. Color change of phenol red and TEAB system with hydrogen peroxide vapor is faster in EC001270 (an acrylate) and slower in Witcobond 213, (a polyurethane of Witco Corporation, Houston, TX). Permeability of the reactive gases can also be controlled by addition of a crosslinking agent and other additives such as plasticizers which change the rate of diffusion. When exposed to hydrogen peroxide vapor, the color change of phenol red with potassium iodide in EC001270 is blue while it is red in polyvinyl alcohol. The pH of the coating of EC001270 is about 4-5 while that of polyvinyl alcohol is about 7.

Though aqueous ink or coating formulations are preferred, one can use solvent based coating formulations polymers used in such formulations are cellulose nitrate, carboxymethyl cellulose, polyolefins, polyvinylchloride, polyurethane, polysilicones and polyepoxy.

**Effect of nature of the polymer:** Coatings prepared from phenol blue as indicator, TEAB as activator and a variety of polymeric materials as binders were exposed to hydrogen peroxide vapor. EC001270 extender was the fastest binder, Witco 160 (a polyurethane of Witco Corporation) and Rovace 571 (a polyvinylacetate latex by Rohm and Haas) were medium and Airflex TL40 (a polyvinylacetate latex by Air Products) was the slowest. They showed the intermediate color, purple before turning blue.

If the barrier coat is of varied thickness, a moving boundary device can be created. Such device was created by applying a wedge shaped coating with a wedge shaped coating bar of EC001270 on the coating of EC001270, phenol red and TEAB. A boundary was created between the original yellow color and the new blue color after about 2 hours when exposed to hydrogen peroxide. The boundary moved from the thin end towards the thick end with prolonged exposure.

**Two-layer device**: The device may have more than one indicator layer, each containing indicator, activator and polymer. In order to get at least more than one color change the indicator should be different in different indicator layers and should undergo different color changes.

Both layers do not have to undergo color changes with plasma. Even if one layer undergoes a color change, a color change can be noticed, especially if the top layer becomes colorless

**Mixtures:** Desired colors, color changes and time required for the color changes can be obtained by mixing proper indicators, activators, additives and polymer in appropriate amounts.

**Multi color changes:** We have found that by mixing more than one dye, each undergoing different color changes, one can obtain more than one color changes. Similarly, one can use a mixture of pigments or dyes which are not affected by plasma and indicators, especially those which become colorless or lighter color with plasma, to get more than one color change. The rate of reaction was also varied by using a mixture of more than one activators of different reactivities.

**Substrate:** Though the device may be self-supporting polymer film containing the activator and indicator, it is desirable to prepare the device on substrate. The device can be made by coating the indicating formulation on a substrate. The substrate could be any solid surface; for example one made from paper, plastic, ceramic or metal. The substrate could be a container, e.g., bag or pouch, for items to be sterilized. The sterilization indicator can also be prepared in form of stickers, tapes and strips.

Although any solid substrate having a smooth surface can be used, a preferred substrate is a flexible and transparent plastic film, natural (cellulose) or synthetic (e.g., spun bonded polyolefins, e.g., Tyvek^{R}) papers. Plastic films, such as polyethylene, polypropylene, polyvinylchloride, polymethylmethacrylate, polyurethanes, nylons, polyesters, polycarbonates, polyvinylacetate, cellophane and esters of cellulose can be used as the transparent substrate. Metal foils, such as aluminum can be used. The most preferred substrates are the 5 - 300 microns thick films of polyethylene terephthalate and Tyvek^{R}.

**UV absorber:** If a plasma indicator formulation is sensitive to ultraviolet light, UV absorbing materials can be added to minimize the effect. A large number of UV absorbers are available commercially, e.g., those used in sun-tan lotions, e.g., Tinuvins of Ciba-Geigy Corporation. UV absorbers include compounds such as maleic acid, sodium salicylate, benzophenone, or benzophenone tetracarboxylate and a large number of polyaromatic compounds.

**Temperature:** The indicator can undergo a color change in a substantial temperature range, namely from a very low temperature (e.g., - 30°C) to a very high temperature of a few hundred degrees centigrade. The preferred temperature is below about 60°C, most preferred temperature is ambient.

**Time:** The time required for color change depends upon the nature of the plasma. A more reactive and more diffusive plasma would change color faster. The time required for the color change can be varied by controlling the diffusion of the components of the plasma by adding proper additives in the polymer. Crosslinking the polymer will reduce the diffusion of the plasma and increase the time required for the color change. Additives which react with plasma can also increase the time required for the color change. Similarly, certain additives, such as oxidant and plasticizer would decrease the time

### 1. Thickness of the polymer indicator layer.

The thickness of the indicator and barrier layers may vary from a micron to a few hundred microns. The preferred thickness is 1-50 microns and the most preferred range is 2-20 microns.

### 2. Concentration of the activator .

The concentration of activator may vary from 0.1 to 50 w/w%. The preferred concentration is 1 to 20 w/w% and the most preferred concentration is 2-10 w/w/%.

### 3. Concentration of the indicator.

The concentration of the indicator may vary from 0.1 to 20w/w%. The preferred concentration is 10 to 10w/w% and the most preferred concentration is 2-5 w/w%.

### 4. Concentration of other additives.

The concentration of additives may vary from 0.1 to 20w/w%. The preferred concentration is 0.5 to 10w/w% and the most preferred concentration is 1-5w/w/%.

### 5. Nature of the barrier

This is the same group as the polymer, but may be different from it

### 6. Thickness of the barrier

The barrier may be between 2 and 200 microns thick, preferrably 2-20 microns.

### 7. Nature of the additives

The preferred time range for sterilization is from 5 minutes to a few hours, The most preferred time is about 15-60 minutes.

In addition to the plasma of hydrogen peroxide, the device may be used with other plasmas, such as that of peracetic acid and a mixture of hydrogen, oxygen and argon.

**Advantages:** The plasma indicator disclosed here offers the following advantages:
* . It is selective to plasma (i.e., no or least effect of other sterilants).
* . It provides desired color changes (from a starting light color, such as white/colorless, yellow, orange, pink, or red to a final dark color, such as blue, green, black, purple or violet).
* . It provides an intermediate color for monitoring a partial cycle.
* . The time, temperature etc required for the color change can be varied by simple means.
* There is essentially no effect of ambient conditions (e.g., dry heat, humidity and light) before and after the sterilization.
* . It is unaffected by sealing hot bar.
* . It has required pot life.
* . There is no bleeding/diffusion of dyes.
* . The ingredients (indicators/dyes and activators/additives) are water soluble. No grinding of ingredients required.
* . Formulations can be made by simple procedures (mixing/dissolution).
* . Ink is printable with gravure and flexo presses on polyester, paper and Tyvek.
* . The print rolls are easy to clean.
* . It uses least toxic or hazardous chemicals.
* . It uses readily available chemicals (dyes, activators and binders).
* . There is an option of precision measurement with moving boundary.
* . Formulations are inexpensive.
* . It is unaffected by ethylene oxide, steam, heat and radiation.

### EXAMPLES

### Example 1. General procedure for preparation of the sample devices.

In a 10ml test tube were added 3 ml of EC001270 (an acrylate ink extender supplied by Environmental Inks and Coating, Co, Lithicum, MD), about 0.5 ml of solution (60 w/w% TEAB in water) and 0.5 ml of a solution (e.g., 4 w/w% solution of a phenol red in ethanol). The contents were mixed and coated with a #10 wire wound rod on a 100 micron polyester film, paper and Tyvek^{R}. The coatings were dried in an oven at about 50°C for 5 minutes.

Experiments 1-8 are not according to the claimed invention.

### Experiment 1: Exposure to vapor of hydrogen peroxide.

Samples of example 1 were hung on the side of a 20-liter dear glass battery jar containing an empty petri dish. About 50 ml of 30% hydrogen peroxide was poured in the petri dish and the jar was tightly closed. The color changes of the samples were noted. For testing under low pressure a vacuum desicator was used instead of the battery jar.

### Example 2: Effect of activator on dyes.

Using the general procedure described in example 1. coatings were prepared from EC001270 as a polymer, TEAB as an activator, and several of the dyes listed in Table 2 as indicators. The coatings were exposed to one sterilization cycle of hydrogen peroxide plasma using STERRAD^{®} model 100, sterilization system made by Advanced Sterilization Products (address). Most of these dyes changed their colors. Some of them either became lighter or colorless while the others developed colors. Only a small number of the dyes showed color change without the activator. Representative color changes are listed Table 4.

**Table 4. Representative color changes of some dyes with ECOO1270 and TEAB**

| **Dye** | **Original color** | **Color after plasma treatment** |
|---|---|---|
| Acid blue 93 | Blue | Light blue |
| Evans blue | Blue | Colorless |
| Thionin | Blue | Red |
| Disperse blue 3 | Blue | Light red |
| Malachite green oxalate | Green | Colorless |
| Disperse red 14 | Purple | Yellow |
| Acid fuschin | Red | Colorless |
| Pararosaniline acetate | Pink | Colorless |
| Dichlorofluorescein | Light pink | Red |
| Brilliant yellow | Yellow | Red |
| m-Cresol purple | Yellow | Purple |
| Phenol red | Yel low | Blue |
| Cresol red | Yellow | Green |
| Fluorexon | Yellow | Red |
| Basic yellow 11 | Yellow | Colorless |
| Fluorescein | Colorless | Red |

Similar color changes were obtained with many other activators, such as sodium nitrite, sodium thiocyanate, salts of amines and acids and other binders such as polyurethane (Witcobond 213, Witco Corporation, Houston, TX) and cellulose nitrate.

### Example 3: Effect of other halides.

Using the procedure described in example 1, coatings were prepared with certain organic and inorganic halides as activators and fluorescein, phenol red, chlorophenol red, acid fuschin, brilliant green and their mixtures as indicators. The coatings were exposed to hydrogen peroxide and its plasma. All coatings of acid fuschin changed from red-to-colorless and that of brilliant green changed from green-to-colorless with plasma. The color changes of the other three dyes are listed in Table 5.

**Table 5**

| Effect of different halides on color changes of some dyes with hydrogen peroxide plasma. | | | |
|---|---|---|---|
| The original color of the coatings was yellow | | | |
| Dye | Fluorescein | Phenol Red | Chlorophenol Red |
| Acetylcholine chloride | Pink | L.blue | L.Yellow-green |
| Ammonium bromide | Pink | Green | L.Yellow-green |
| Choline chloride | Orange | Colorless | L.Yellow-green |
| Tetrabutyl ammonium bromide | Pink | Yellow | L.Yellow-green |
| Choline iodide | Orange | Colorless | L.green |
| Potassium bromide | Pink | Blue | L.green |
| Potassium iodide | Yellow | Yellow | Yellow |
| Sodium iodide | Yellow | Yellow | Yellow |
| Tetraethylammonium bromide | Pink | Blue | Blue green |
| Tetrahexylammonium bromide | Yellow | Yellow | Yellow |
| Tetramethyl ammonium acetate | Orange | Blue | L.blue |

| | | | |
|---|---|---|---|
| L. = light | | | |

Mixtures of halides also provided essentially the same color changes. Bromides were more effective than other halides in introducing the color change. The iodides show color change when polyvinyl alcohol is used as a binder.

### Example 4: Effect of bromides.

Using the general procedure described in example 1, coatings were prepared using phenol red as an indicator and several organic and inorganic bromides e.g., ammonium bromide, barium bromide, calcium bromide, iron (III) bromide, potassium bromide, zinc bromide, diphenyliodinium bromide, tetrabutylammonium bromide, tetraethylammonium bromide, tetrahexylammonium bromide, dimethyldioctadecylammonium bromide and phenacylpyridinium bromide and mixtures of some bromides as activators. The coatings were exposed to hydrogen peroxide vapor and its plasma.

All coatings changed from yellow-to-blue. Coatings containing dimethyldioctadecylammonium bromide changed slowly while those of ammonium bromide, potassium bromide and tetraethylammonium bromide changed faster.

### Experiment 2 :_ Effect of pH of the polymer medium.

The pH of the EC001270 coating is about 4-5 while that of polyvinyl alcohol (PVOH) is about 7. Coating were prepared using potassium iodide and TEAB as activators and brilliant yellow, m-cresol purple, cresol red and phenol red as indicators in EC001270 and polyvinylalcohol as binders. The coatings were exposed to hydrogen peroxide vapor. The coatings of KI/PVOH changed colors in seconds. The results indicate that the device can be used for monitoring hydrogen peroxide. As shown in Table 6, depending upon the pH of the binders, different color can be obtained.

**Table 6. Color of some dyes with different polymer and activators.**

| Dye | Polymer Activator | ECOD1270 TEAB | PVOH TEAB | EC001270 KI | PVOH KI |
|---|---|---|---|---|---|
| Brilliant yellow | | Light red | Yellow | Light orange | Red |
| m-Cresol purple | | Blue | Light orange | Orange | Blue |
| Cresol Red | | Light blue | Yellow | Blue | Blue |
| Phenol Red | | Blue | Yellow | Yellow | Purple |

Effect of nature of polymer: The other polymers were much slower, e.g., took more than ten hours compared to less than hour for EC.

### Experiment 3: Effect of a barrier coat.

A coating of EC001270, phenol red and TEAB bromide of example 1 was top-coated with EC001270 using number 3, 5, and 10 wire wound rods. After drying, the coatings were exposed to hydrogen peroxide vapor. The time required for the color change is shown in the table 7.

**Table 7. Effect of thickness of a barrier coat on the time required for the color change.**

| Top-coating Bar | Time (hours) required for the color change |
|---|---|
| None | 0.1 |
| 3 | 3 |
| 5 | 7 |
| 10 | 18 |

### Experiment 4 : Two layer device, both containing indicators.

coating of EC001270, phenol red and TEAB was top-coated with that of EC001270, pararosaniline acetate and TEAB. This double-coated device appeared red. The device was exposed to hydrogen peroxide vapor. Pararosaniline of the top-coat changed from red-to-colorless. Phenol red of the undercoat then changed from yellow-to-blue. The color changes of the device with time are shown in Table 8.

**Table 8. Color changes of the two-layer device having phenol red in the undercoat and pararosaniline in the top-coat.**

| | | | | | |
|---|---|---|---|---|---|
| Time (hours) | 0 | 3 | 6 | 10 | 24 |
| Color | Red | Ught red | Purple | Blue | Dark blue |

### Experiment 5 : Two-layer device, one containing indicator.

The two layer device was prepared by first coating a red pigmented ink followed by a top-coating of EC001270, malachite green oxalate and TEAB. The resultant device appeared very dark, almost black The device was exposed to hydrogen peroxide. The device changed from black-to-red in about six hours. The color change is due to fading of the green color of malachite green oxalate which make the red color of the under coat visible.

### Experiment 6 : Moving boundary device.

A moving boundary device was created by applying a wedge shaped coating with a wedge shaped coating bar (0 at one end and #10 at the other end) of EC001270 on the coating of EC001270, phenol red and TEAB. The device thus created was exposed to hydrogen peroxide vapor. A boundary was created between the original yellow color and the new blue color after about 2 hours. The boundary moved from the thin end towards the thick end. The distance traveled by the boundary is shown in Table 9.

**Table 9. Distance traveled bv the moving boundary device**

| | | | | |
|---|---|---|---|---|
| Time (hours) | 2 | 4 | 10 | 24 |
| Distance (cm) | 0.2 | 0.5 | 2 | 3 |

### Experiment 7: Detection of residual absorbed hydrogen peroxide.

A piece of 100-micron polyester film having a coating of EC001270 was exposed to hydrogen peroxide vapor for two hours. The coated film was removed and hung in air for ten minutes. A coating of polyvinyl alcohol, m-cresol red, and Kl coated on polyester film was placed on hydrogen peroxide exposed film. The yellow coating turned light blue within 15 minutes.

In accordance with the above procedure, the device may be used for the detection of ozone and other oxidative vapors.

### Example 5: Pilot coating.

In 1200g EC001270 extender the following were added while stirring: 18g of 28% ammonium hydroxide, 100g of water, 200ml of isopropanol, 36g pararosaniline acetate, 18g of phenol red and 64g of TEAB. The formulations were coated on Tyvek^{R} and polyester film using a pilot coater of Rexam Medical Packaging, Mt. Holly, NJ. Eleven other formulations with different ratios of the dyes, TEAB and crosslinking agent (a polyaziridine) were also coated. The coatings were red (burgundy) color.

The samples were treated with half, one and two cycles of plasma sterilization cycles of hydrogen peroxide plasma using STERRAD^{®} model 100, sterilization system of Advanced Sterilization Products. The samples treated with half cycle were yellow-green while those treated with one and two cycles were blue.

The coated samples were exposed to 100% ethylene oxide and steam sterilization cycle. We found that there was no or negligible effect of (1) 100% ETO for 16 hours and (2) normal steam sterilization treatment (e.g., 20 minutes at 121°C). Steam makes the color a bit lighter and ETO makes slightly purple/violet

The samples were annealed at 60°C for five days and exposed to 100% humidity at 60°C for 5 days and then treated with the plasma along with control (un-treated samples) for determination of the shelf life and color stability. We found that there was no or negligible effect of: (1) dry heat at 60°C five days and (2) 100% humidity at 60°C for five days.

Samples treated with half, one and two cycles of the plasma were annealed at 60°C for five days and exposed to 100% humidity at 60°C for 5 days for determination archival life and color stability. There was no or negligible effect of (1) dry heat at 60°C five days, (2) 100% humidity at 60°C for five days on the final blue color.

The effect of hydrogen peroxide vapor depends on the exposure time. There was essentially no color change for about 4 hours. After about 5 hours the samples started turning yellow. At about 8 hours it started turning green color and at about 16 hours, they turned blue.

### Example 6 ; Alternate embodiment

A coating of EC001270, phenol red and TEAB was top-coated with that of EC001270, pararosaniline acetate and TEAB. This double-coated device appeared red. The device was exposed to hydrogen peroxide vapor and its plasma. First, pararosaniline in the top-coat changed from red-to-colorless. Phenol red in the undercoat then changed from yellow-to-blue.

### Experiment 8 : Alternate embodiment

An alternate structure of this type of device was prepared by first coating a red pigmented ink followed by a top-coating of EC001270, malachite green oxalate and TEAB. The resultant device appeared very dark, almost black. The device changed from black-to-red when exposed to hydrogen peroxide.

To get orange-to-yellow and red/burgundy-to-yellow, methyl red was mixed with cresol red and add fuschin with cresol red along with TEAB as activator and EC001270 as a polymer. Red-to-blue color was obtained by mixing pararoseaniline acetate and phenol red as shown in example 5.

### Example 7 : Alternate embodiment

A mixture of pararoseaniline acetate and phenol red in the EC extender with tetraethylammonium bromide as an activator provides burgundy-to-green-to-blue color changes when treated with hydrogen peroxide plasma.

To get orange-to-yellow and red/burgundy-to-yellow, methyl red was mixed with cresol red and acid fuschin with cresol red along with TEAB as activator and EC001270 as a polymer.

## Claims

1. A device for monitoring plasma comprising:
at least one layer of polymer exposable to said plasma, having incorporated therein
a) an indicator capable of undergoing at least one color change
b) an activator for said indicator wherein said activator, when contacted with said plasma, undergoes a reaction wherein the product of said reaction causes said indicator to undergo said color change.

2. The device of claim 1 wherein the said indicator comprises at least one member of the group consisting of pigments, dyes, precursors of said dyes, and mixtures of any of the foregoing group members.

3. The device of claim 1 and/or 2 wherein the said indicator is a pH-sensitive dye.

4. The device of any of claims 1 - 3 wherein said polymer is soluble in an organic solvent.

5. The device of any of claims 1 - 3 wherein said polymer is soluble in water or is water dispersible.

6. A process of making a device of any of claims 1 - 5 which comprises dissolving or dispersing the components thereof in a solvent therefore, applying the thus formed solution or dispersate to a substrate and permitting the solvent to evaporate.

7. A process of using a device of any of claims 1 - 5 for monitoring sterilization of materials comprising the steps of
a) affixing the device to said materials or containers containing same
b) carrying out the process of sterilization including the step of introducing the plasma into a vessel containing said materials or containers therefore
and
c) observing the presence of a color change of said device.

8. The process of claim 7 wherein the plasma is derived from a member selected from the group consisting of hydrogen peroxide, perchloric acid and oxygen.

9. A process of using the device of any of claims 1 - 5 for monitoring an oxidizing plasma comprising the steps of
a) exposing the device to an oxidizing plasma,
b) observing the presence of color change in the device.

10. The process of claim 9 wherein the plasma is that derived from hydrogen peroxide.

## Patentansprüche

1. Vorrichtung zur Überwachung von Plasma, enthaltend:
wenigstens eine Polymerschicht die dem Plasma ausgesetzt werden kann, in die eingearbeitet ist
a) ein Indikator, der wenigstens eine Farbänderung durchmachen kann,
b) einen Aktivator für den Indikator, wobei der Aktivator, wenn er mit dem Plasma in Kontakt gebracht ist, einer Reaktion unterliegt, worin das Produkt der Reaktion verursacht, dass der Indikator die Farbänderung durchmacht.

2. Vorrichtung nach Anspruch 1, wobei der genannte Indikator wenigstens ein Glied der aus Pigmenten, Farbstoffen, Vorläufern für die Farbstoffe und Gemischen aus beliebigen Mitgliedern der vorgenannten Gruppe besteht.

3. Vorrichtung nach Anspruch 1 und/oder 2, wobei der Indikator ein pH-empfindlicher Farbstoff ist.

4. Vorrichtung nach einem der Ansprüche 1 bis 3, wobei das Polymer in einem organischen Lösungsmittel löslich ist.

5. Vorrichtung nach einem der Ansprüche 1 bis 3, wobei das Polymer in Wasser löslich oder dispergierbar ist.

6. Verfahren zur Herstellung einer Vorrichtung nach einem der Ansprüche 1 bis 5, dass das Auflösen oder Dispergieren seiner Komponenten in einem Lösungsmittel hierfür, dass Aufbringen der so gebildeten Lösung oder Dispersion auf ein Substrat und das Abdampfenlassen des Lösungsmittels umfasst.

7. Verfahren zur Anwendung einer Vorrichtung nach einem der Ansprüche 1 bis 5 zur Überwachung der Sterilisation von Materialien, umfassend die Schritte:
a) Anbringen der Vorrichtung an die Materialien oder Behälter, die dieselben enthalten,
b) Durchführen des Sterilisationsverfahrens einschließlich der Schritte des Einführens des Plasma in ein Gefäß, das die Materialien enthält, oder Behälter hierfür und
c) Beobachtung des Vorliegens einer Farbänderung der Vorrichtung.

8. Verfahren nach Anspruch 7, wobei das Plasma aus einem Mitglied der aus Wasserstoffperoxyd, Perchlorsäure und Sauerstoff bestehenden Gruppe ausgewählt ist.

9. Verfahren zur Anwendung der Vorrichtung nach einem der Ansprüche 1 bis 5 zur Überwachung eines oxidierenden Plasmas, enthaltend die Schritte:
a) Man setzt die Vorrichtung einem oxidierenden Plasma aus,
b) Man beobachtet das Vorliegen einer Farbänderung in der Vorrichtung.

10. Verfahren nach Anspruch 9, wobei das Plasma dasjenige ist, das von Wasserstoffperoxyd abstammt.

## Revendications

1. Dispositif pour surveiller un plasma comprenant :
au moins une couche de polymère pouvant être exposée audit plasma, dans laquelle sont incorporés
a) un indicateur capable de subir au moins un changement de couleur
b) un activateur pour ledit indicateur où ledit activateur, quand il est mis en contact avec ledit plasma, subit une réaction où le produit de ladite réaction amène ledit indicateur à subir ledit changement de couleur.

2. Dispositif selon la revendication 1 où ledit indicateur comprend au moins un membre du groupe consistant en les pigments, les colorants, les précurseurs desdits colorants, et les mélanges de membres quelconques du groupe précédent.

3. Dispositif selon la revendication 1 et/ou 2 où ledit indicateur est un colorant sensible au pH.

4. Dispositif selon l'une quelconque des revendications 1 - 3 où ledit polymère est soluble dans un solvant organique.

5. Dispositif selon l'une quelconque des revendications 1 - 3 où ledit polymère est soluble dans l'eau ou est dispersible dans l'eau.

6. Procédé pour produire un dispositif selon l'une quelconque des revendications 1 - 5 qui comprend la dissolution ou la dispersion de ses composants dans un solvant pour eux, l'application de la solution ainsi formée ou du produit de dispersion ainsi formé à un substrat et l'évaporation du solvant.

7. Procédé d'utilisation d'un dispositif selon l'une quelconque des revendications 1 - 5 pour surveiller la stérilisation de produits comprenant les étapes de
a) fixation du dispositif auxdits produits ou aux récipients qui les contiennent
b) mise en oeuvre du procédé de stérilisation incluant l'étape d'introduction du plasma dans un vase contenant lesdits produits ou des récipients pour eux
et
c) l'observation de la présence d'un changement de couleur dudit dispositif.

8. Procédé selon la revendication 7 où le plasma est dérivé d'un membre choisi dans le groupe consistant en le peroxyde d'hydrogène, l'acide perchlorique et l'oxygène.

9. Procédé d'utilisation du dispositif selon l'une quelconque des revendications 1 - 5 pour surveiller un plasma oxydant comprenant les étapes de
a) exposition du dispositif à un plasma oxydant,
b) observation de la présence d'un changement de couleur dans le dispositif.

10. Procédé selon la revendication 9 où le plasma est celui dérivé du peroxyde d'hydrogène.
